# EUROPEAN PATENT APPLICATION

(11) **EP 4 202 946 A1**
(43) Date of publication of application: **28.06.2023**
(21) Application number: 21306889.3
(22) Date of filing: 21.12.2021
(51) Int. Cl.: G16H 30/00

(54) **METHOD AND SYSTEM FOR TRACKING THE EVOLUTION OF A WOUND**

(71) Applicant: Bull SAS, 78340 Les Clayes-sous-Bois (FR)
(72) Inventor: BLAZSOVSKY, Fiona, 1090 VIENNA (AT)
(74) Representative: IPAZ

(57) **Abstract**

The invention relates to a method (100) for tracking the evolution of a wound, comprising the following steps:
- capturing (102) an initial picture of a patient's wound with a user equipment comprising a camera, and
- analysing (104) the type of the wound by a pre-trained neural network,
characterised in that said method also comprising at least one iteration of a step of monitoring (106) the wound over time comprising the following steps:
- capturing (108) at least one new picture of said patient's wound, and
- comparing (110) said new picture to at least one previous picture.

## Description

The present invention relates to a method and a system for tracking the evolution of a wound.

The field of the invention is the field of wounds evolution tracking methods and systems.

### Background

In order to track the evolution of a wound of a patient, practitioners are forced to rely on notes taken during a previous examination or worse rely on their own memory of a previous examination.

Using examination notes for tracking of the evolution of a patient's wound is inherently subjective and therefore might not be reliable. Also, the use of examination notes for tracking of the evolution of a patient's wound makes it difficult to track small evolution of a wound, when the patient's wound heals or deteriorates at a slow rate and the changes of the wound between consultation are hard to distinguish.

Additionally, relying on notes and/or memory of the examinations makes it harder for a practitioner to take over the tracking of the evolution of a patient's wound from another practitioner.

A purpose of the present invention is to overcome at least one of these drawbacks.

Another purpose of the invention is to propose a more accurate method and system for tracking the evolution of a wound.

Yet another purpose of the present invention is to propose a method and a system for tracking the evolution of a patient's wound easier to implement by different practitioners over time.

### Summary of the invention

The invention makes it possible to achieve at least one of these aims by a method for tracking the evolution of a wound, comprising the following steps:
- capturing an initial picture of a patient's wound with a user equipment comprising a camera,
- analysing the type of the wound by a pre-trained neural network, characterised in that said method also comprising at least one iteration of a step of monitoring the wound over time comprising the following steps:
- capturing at least one new picture of said patient's wound,
- comparing said new picture to at least one previous picture.

Thus, the present invention provides a method to track of the evolution of a patient's wound where an initial picture of a wound is taken, the wound's type is analysed and the wound is monitored over time by comparing new pictures to previously taken picture.

Consequently, the invention provides a method that is a less subjective as it relies on pictures taken over time rather than on practitioner's notes. Since a practitioner's note might change based on his perception and personal experience, the method provides a more reliable tracking of the evolution of a patient's wound.

Moreover, the invention provides a more accurate method for tracking the evolution of patient's wound. Indeed, pictures accurately capture the state of the wound at the time at which it was taken. Therefore, it is much more accurate to monitor the wound over time by comparing a new picture of a patient's wound to a picture previous taken as it allows to identify even the slightest evolution of the wound that might have gotten lost using only notes.

Furthermore, the invention provides a method to track the evolution of a patient's wound where the monitoring of a patient wound over time can more conveniently be carried out by different practitioners. Indeed, said monitoring relies on previously taken pictures and does not rely on a previous practitioner's notes and therefore can easily be taken over by another practitioner.

### Advantageously:

- the method can comprise, prior to the analysing step, a step for gathering clinical data from health records of said patient, and
- the analysing of the type of the wound by a pre-trained neural network can use said captured picture and said gathered clinical data.

Thus the present invention provides a method that can analyse the type of wound more accurately.

Indeed, a neural network pre trained to use a picture of a patient's wound jointly with clinical data can be tuned more finely and provide a better analysis of the type of wound captured in the picture.

The gathered clinical data can for example comprise but are not limited to:
- patient history,
- patient data, such as his age, gender or ethnicity,
- geographical location,
- location of the wound,
- etc...

Advantageously, the analysing step, respectively the comparing step, can be carried out on said user equipment.

Thus, the method can be carried out using a single user equipment. Therefore, it is possible to track and monitor the evolution of a wound over time using an all-in-one solution that does not require access to internet or any other external resources. It is therefore possible to monitor the evolution of a wound over time even when said patient is located on in a remote area or even a war zone where access to external resources are scarce.

Alternatively:
- the analysing step, respectively the comparing step, can be carried out on a server distant from said user equipment, and
- the method can comprise at least one upload step, to upload said initial picture and said at least one new picture to said distant server.

Thus, the present invention provides a method that can be carried out using a less powerful user equipment, and therefore a cheaper user equipment, as the steps requiring the most processing power are carried out on a distant server.

Therefore, the user equipment just requires to be able to take pictures and upload said pictures to a distant server. The method can therefore be more accessible to users, especially in poorer countries where users might not have access to powerful user equipment.

Additionally, the use of a distant server makes it possible to access results from the analysing step respectively the comparing step for a distance, making it in turn easier for a practitioner to monitor the evolution of a patient's wound from a distance.

Advantageously, the pre-trained neural network can be a convolutional neural network.

In an embodiment of the invention, particularly in an embodiment where the comparing step is carried out using a user equipment, the comparing step can be carried out visually by displaying on the user equipment the new picture and a previously taken picture, for example displayed side by side.

Advantageously, the comparing step can be carried out by an image comparison software.

Thus, the present invention provides a more accurate monitoring of a patients wound over time as an image comparison software can detect and compare characteristics of patients wound more accurately.

The characteristics of a patients wound compared by comparison software can for example comprise but not limited to:
- wound size,
- wound colour,
- wounds shape,
- wound surface area,
- wound depth,
- etc...

In a preferred embodiment, at least one reference item, such as a ruler or a colour chart, is comprised in each of the picture of a wound, alongside the wound to facilitate comparison of the pictures.

In an embodiment where each picture comprises a reference ruler, the sizing of a wound can be done more precisely by the comparison software, for example by using digital planimetry.

Alternatively, or in addition, the comparison software can automatically identify some reference points comprised in every picture of a patient's wound. As non-limitative examples, the reference points can be a mole, a scare, or any other feature of the patient's anatomy.

In a further aspect the invention provides a system for tracking the evolution of a wound, comprising:
- at least one user equipment comprising a camera, for capturing an initial picture of a patient's wound and, over time, capturing at least one new picture of a patient's wound, and
- at least one application for:
   ▪ analysing the type of the wound using a pre-trained neural network, and
   ▪ comparing said new picture to at least one previous picture to monitor the wound over time.

Advantageously, said at least one application for analysing the type of wound and comparing captured pictures can be located on said at least one user equipment.

Thus, the system can be comprised in a single user equipment. Therefore, it is possible to track and monitor the evolution of a wound over time using an all-in-one solution that does not require access to internet or any other external resources. It is therefore possible to monitor the evolution of a wound over time even when said patient is located on in a remote area or even a war zone where access to external resources are scarce.

Advantageously, a single application for analysing the type of wound and comparing captured pictures can be located on said at least one user equipment.

Thus, different step for tracking the evolution of a patient's wound are streamlined within a single application. This makes tracking the evolution of a patient's wound more user friendly.

Alternatively:
- the system comprises a server distant from said at least one user equipment,
- said at least one application for analysing the type of wound and comparing captured pictures are located on said server.

Thus, the system can use less powerful user equipment, and therefore cheaper user equipment, as the applications requiring the most processing power are carried out on a distant server.

Advantageously, a single application, called client application, can be located on the user equipment, such that the client application can capture picture of a wound and the upload said captured picture to a distant server.

Said at least said at least one user equipment can be a smartphone, or a personal computer.

### Description of the figures and embodiments

Other advantages and characteristics will become apparent on examination of the detailed description of an embodiment which is in no way limitative, and the attached figures, where:
- Figure 1 is a schematic representation of a non-limitative example of a method for tracking the evolution of a wound according to the invention;
- Figure 2 is a schematic representation of another non-limitative example of a method for tracking the evolution of a wound according to the invention;
- Figure 3 is a schematic representation of another non-limitative example of a method for tracking the evolution of a wound according to the invention;
- Figure 4 is a schematic representation of a non-limitative example of a system for tracking the evolution of a wound according to the invention; and
- Figure 5 is a schematic representation of another non-limitative example of a system for tracking the evolution of a wound according to the invention.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the figures, elements common to several figures retain the same reference.

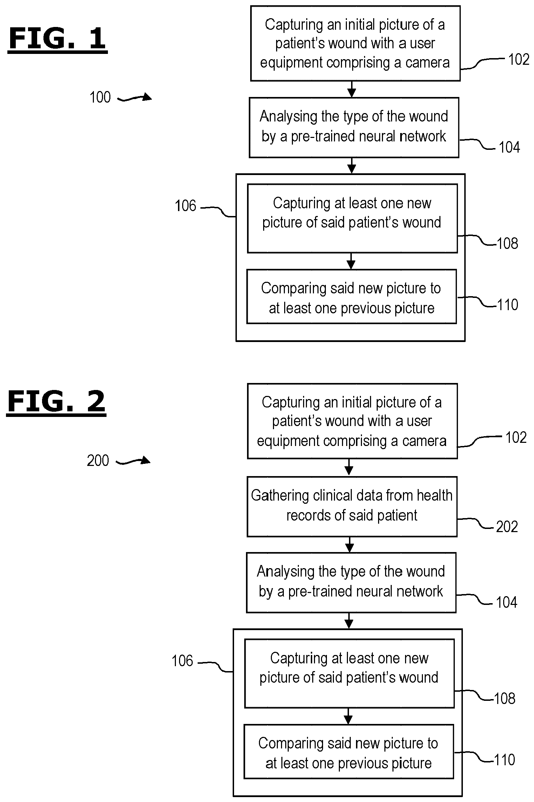

Figure 1 is a schematic representation of a non-limitative example of a method for tracking the evolution of a wound according to the invention.

The method 100 for tracking the evolution of a wound, represented in FIGURE 1, comprises a step 102, capturing an initial picture of a patient's wound with a user equipment comprising a camera.

The initial picture of a patients wound can be taken by any user, such as for example, the patient himself or by a practitioner.

Preferentially, at least one reference item, such as a ruler or a colour chart, can be placed next to the wound prior to the capturing step 102 such that the captured initial picture of the patient's wound also comprises said reference item. Said reference item can make it easier to size and/or colour grade the wound making future comparison potentially easier.

The method 100 comprises, after the capturing step 102, a step 104 for analysing the type of the wound by a pre-trained neural network. During said step, the captured initial picture, the type of wound can be identified and classified. The type of wound can for example and non-imitatively be: a cut, a burn, an infection, a bite, a chronic wound, etc...

The neural network may a convolutional neural network trained, by a training algorithm, on training data base comprising training data sets.

The training algorithm may be the backpropagation algorithm optimizing a cost function.

Each training data set may comprise an image of a wound and a label indicating the type of the wound.

The method 100 comprises at least one iteration of a step 106 of monitoring the wound over time. Said monitoring step 106 comprises a step 108 for capturing at least one new picture of said patient's wound followed by a step 110 for comparing said new picture to at least one previous picture.

The new picture of a patients wound can be captured by any user, such as for example, the patient himself or by a practitioner.

Preferentially, the same reference item as the one placed for the initial picture can be placed next to the wound prior to the capturing step 108 such that the new captured picture of the patient's wound also comprises said reference item. Said reference item makes it easier to compare the wound captured in the new picture and the wound in the initial picture.

The picture to which said new picture is compared to during the comparing step 110 can be said initial picture of said patient's wound or any picture of said patient's wound taken during a prior iteration of the monitoring step 106, preferentially taken during the latest prior iteration of a monitoring step 106.

The comparison step 110 can be done visually, for example by displaying the new picture and the picture it is compared to side by side on a user equipment for a practitioner to compare.

In an alternative preferred embodiment, the comparison step can be done using an image comparison software that can detect and compare characteristics of patients wound less subjectively and more accurately. The comparison software can for example compare wound characteristics such as:
- wound size,
- wound colour,
- wounds shape,
- wound surface area,
- wound depth,
- etc...

The comparison may indicate whether the wound is healing or not. For example, the comparison may indicate the evolution of the size, or the depth of the wound.

Figure 2 is a schematic representation of another non-limitative example of a method for tracking the evolution of a wound according to the invention.

The method 200 illustrated in FIGURE 2 comprises all the steps of the method 100 described above in relation with FIGURE 1.

The method 200 also comprises, after the step 102 for capturing an initial picture of a patient's wound and prior to the step 104 for analysing the type of wound, a step 202 for gathering clinical data from health records of said patient.

Said clinical data gathered during step 202 can then be used by a pre-trained neural network, jointly with the captured initial picture, during the step 104 for analysing the type of wound.

In the method 200, the neural network used is therefore pre-trained to use a picture and a clinical data to analyse the type of wound. In this case, each training data set may also comprise training clinical data.

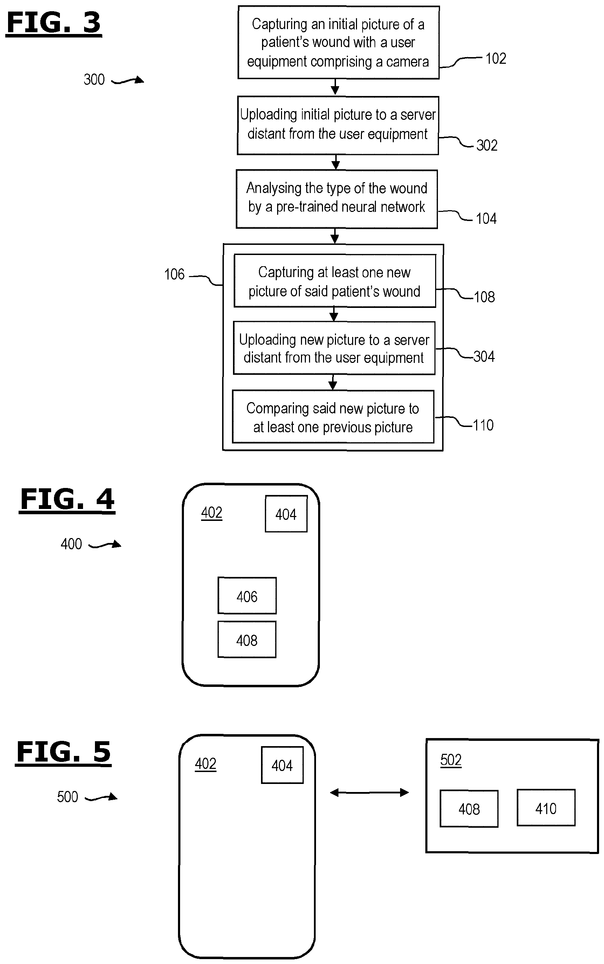

Figure 3 is a schematic representation of another non-limitative example of a method for tracking the evolution of a wound according to the invention.

The method 300 illustrated in FIGURE 3 comprises all the steps of the method 100 described above in relation with FIGURE 1.

The method 300 also comprises, after the step 102 for capturing an initial picture of a patient's wound and prior to the step 104 for analysing the type of wound, a step 302 for uploading the initial picture of a wound to a server distant from the user equipment.

In method 300 the step 104 for analysing the type of wound is carried out on said distant server.

The method 300 also comprises, for each iteration of the monitoring step 106, after the step 108 for capturing a new picture of a patient's wound and prior to the step 110 for comparing said new picture to at least one previous picture, a step 304 for uploading the new picture of a wound to a server distant from the user equipment.

In method 300, for each iteration of the monitoring step 106, the step 110 comparing said new picture to at least one previous picture is carried out on said distant server.

Figure 4 is a schematic representation of a non-limitative example of a system for tracking the evolution of a wound according to the invention.

The system 400 illustrated in FIGURE 4 comprises a user equipment 402 comprising a camera 404, for capturing an initial picture of a patient's wound and, over time, capturing at least one new picture of a patient's wound. The user equipment can for example be a smartphone, a personal computer, a tablet etc...

The system 400 also comprises:
- an application 406 for analysing the type of the wound using a pre-trained neural network, and
- an application 408 for comparing said new picture to at least one previous picture to monitor the wound over time.

In the embodiment of the system 400 illustrated in FIGURE 4, the application 406 for analysing and the application 408 for comparing are located on said user equipment 402.

In another non-illustrated embodiment, the applications 406, 408 and 410 can be combined into a single application.

Figure 5 is a schematic representation of another non-limitative example of a system for tracking the evolution of a wound according to the invention.

The system 500 illustrated in FIGURE 5 comprises a user equipment 402 comprising a camera 404, for capturing an initial picture of a patient's wound and, over time, capturing at least one new picture of a patient's wound.

The system 500 also comprises:
- the application 406 for analysing the type of the wound using a pre-trained neural network, and
- the application 408 for comparing said new picture to at least one previous picture to monitor the wound over time.

In addition, the system 500 comprises a server 502 distant from the user equipment.

In the embodiment of the system 500 illustrated in FIGURE 5, the application 406 for analysing and the application 408 for comparing are located on said distant server 502.

Of course, the invention is not limited to the examples detailed above. For example, the security system of the pruning shears of the present invention may comprise several emitters, and/or several receivers.

## Claims

1. A method (100;200;300) for tracking the evolution of a wound, comprising the following steps:
- capturing (102) an initial picture of a patient's wound with a user equipment (402) comprising a camera,
- analysing (104) the type of the wound by a pre-trained neural network,
**characterised in that** said method also comprising at least one iteration of a step of monitoring (106) the wound over time comprising the following steps:
- capturing (108) at least one new picture of said patient's wound,
- comparing (110) said new picture to at least one previous picture.

2. The method (200) according to any of the preceding claims, **characterized in that**:
- the method (200) comprises, prior to the analysing step (104), a step (202) for gathering clinical data from health records of said patient, and
- the analysing (104) of the type of the wound by a pre-trained neural network uses said captured picture and said gathered clinical data.

3. The method (100;200;300) according to any of the preceding claims, **characterized in that** the analysing step (104) respectively the comparing step (110) are carried out on said user equipment (402).

4. The method (300) according to claim 1 or 2, **characterized in that**:
- the analysing step (104) respectively the comparing step (110) are carried out on a server (502) distant from said user equipment, and
- the method (300) comprises at least one upload step (302,304), to upload said initial picture and said at least one new picture to said distant server (502).

5. The method (100;200;300) according to any of the preceding claims, **characterized in that** the pre-trained neural network is a convolutional neural network.

6. The method (100;200;300) according to the preceding claim, **characterized in that** the comparing step is carried out by an image comparison software.

7. A system (400;500) for tracking the evolution of a wound, comprising:
- at least one user equipment (402) comprising a camera (404), for capturing an initial picture of a patient's wound and, over time, capturing at least one new picture of a patient's wound, and
- at least one application (406,408) for:
▪ analysing the type of the wound using a pre-trained neural network, and
▪ comparing said new picture to at least one previous picture to monitor the wound over time.

8. The system (400) according to the previous claim, **characterized in that** said at least one application (406,408) for analysing the type of wound and comparing captured pictures are located on said at least one user equipment (402).

9. The system (500) according to claim 7, **characterized in that**:
- the system comprises a server (502) distant from said at least one user equipment,
- said at least one application (406,408) for analysing the type of wound and comparing captured pictures are located on said server (502).

10. The system according to any of claim 7 to 9, **characterized in that** said at least said at least one user equipment is a smartphone or a personal computer.
